# EUROPEAN PATENT SPECIFICATION

(11) **EP 1 739 426 B1**
(45) Date of publication and mention of the grant of the patent: **28.10.2009**
(21) Application number: 05728699.9
(22) Date of filing: 01.04.2005
(51) Int. Cl.: G01N 33/543

(54) **IMMUNOLOGICAL ASSAY METHOD AND REAGENT**
IMMUNOLOGISCHES TESTVERFAHREN UND REAGENS
PROCÉDÉ DE DOSAGE IMMUNOLOGIQUE ET RÉACTIF

(30) Priority: 21.04.2004 JP 2004125985; 09.07.2004 JP 2004202807; 06.08.2004 JP 2004231176
(43) Date of publication of application: 03.01.2007
(73) Proprietor: A & T Corporation, Fujisawa-shi, Kanagawa 252-0816 (JP)
(72) Inventor: KAWANO, Mitsuyasu, Fujisawa-shi, Kanagawa 252-0816 (JP); IWAMOTO, Hisahiko, Fujisawa-shi, Kanagawa 252-0816 (JP)
(74) Representative: Albrecht, Thomas
(86) International application number: PCT/JP2005/006893
(87) International publication number: WO 2005/103701

(56) References cited:
- JP-A- 7 306 204
- JP-A- 8 211 061
- JP-A- 62 030 963
- JP-A- 2002 340 890
- JP-A- 2003 057 243
- US-A- 5 236 826
- WOLF-ROGERS J ET AL: "A CHEMILUMINESCENT MICROPARTICLE-MEMBRANE CAPTURE IMMUNOASSAY FOR THE DETECTION OF ANTIBODY TO HEPATITIS B CORE ANTIGEN" JOURNAL OF IMMUNOLOGICAL METHODS, vol. 133, no. 2, 1990, pages 191-198, XP002447442 ISSN: 0022-1759
- FIORE M ET AL: "THE ABBOTT IMXTM AUTOMATED BENCHTOP IMMUNOCHEMISTRY ANALYZER SYSTEM" CLINICAL CHEMISTRY, AMERICAN ASSOCIATION FOR CLINICAL CHEMISTRY, WASHINGTON, DC, US, vol. 34, no. 9, 1 September 1988 (1988-09-01), pages 1726-1732, XP000566918 ISSN: 0009-9147

## Description

### TECHNICAL FIELD

The present invention relates to an immunoassay and a reagent. More specifically, it relates to an immunoassay capable of increasing sensitivity in a short reaction time and a reagent.

### BACKGROUND ART

As a heterogeneous immunoassay of the prior art, JP-A 7-306204 discloses an immunoassay in which fine particles having a ligand bound thereto are dispersed in a liquid, the resulting dispersion is dropped on a porous matrix to capture them, a specimen including a substance to be measured is dropped on the porous matrix capturing the fine particles, a labeled ligand to which a marker is bound is further dropped on the matrix to form an immune complex, and the marker contained in the immune complex is measured. This method includes the step of carrying out a homogeneous reaction for forming the immune complex on the porous matrix twice and the formation of the immune complex is unsatisfactory, thereby making it impossible to increase sensitivity.

JP-A 60-250257 discloses an immunoassay in which a substance binding specifically to a ligand is immobilized to a solid phase and reacted with a specimen to carry out a reaction among biotin, the labeled ligand and the specifically binding substance and further with labeling anti-biotin, an unreacted reagent is separated, and the amount of the marker contained in the solid phase or the unreacted reagent is measured to detect the amount of the ligand existent in the specimen.

Since the substance binding specifically to the ligand is immobilized to the solid phase in the above method, two reactions after that, that is, a reaction between the biotin marker and the substance binding specifically to the ligand and a reaction between the marker and anti-biotin are carried out in a heterogeneous system. Therefore, like the method disclosed by JP-A 7-306204, the formation of the immune complex is unsatisfactory and sensitivity cannot be increased to the full.

Further, JP-A 4-318462 discloses a method of measuring a solid-phase biologically specific reaction, comprising the steps of preparing a porous matrix in which a first substance reactive specifically with a substance to be measured is bonded to a glass fiber filter, supplying a specimen containing the substance to be measured, the substance to be measured to which a detectable signal generating substance is bound or a second substance which reacts specifically with the first substance and a cleaning liquid onto the porous matrix, and measuring the signal generating substance contained in an immune complex remaining on the porous matrix to obtain the amount of the substance to be measured.

The above method involves the same disadvantage as the method of JP-A 7-306204 which includes the step of carrying out a heterogeneous reaction for forming an immune complex on the glass fiber filter twice.

JP-A 2001-235471 also discloses an immunoassay including the step of carrying out a heterogeneous reaction for forming an immune complex on a porous filter though it differs from JP-A 4-318462 in reaction process.

Further, the porous matrix such as the glass fiber filter in the above method is treated with a block solution to suppress a non-specific reaction before use. Since a conventionally known block solution comprises a phosphoric acid buffer physiologic saline as a base material, a non-specific reaction hardly occurs in a certain type of immunoassay and repeatability thereby deteriorates.

Meanwhile, an immune complex is forcedly sucked from below a filter such as a glass fiber filter to be captured with the filter (JP-A 6-148184, JP-A 6-258322, JP No. 2935965 and JP No. 3134231). However, in this conventional suction method, as a liquid passes through the filter quickly, it is difficult to capture the immune complex with the filter surely, or a pressure sensor must be provided to prevent a failure of capture.

U.S. Patent No. 5 236 826 relates to an immunoassay method for the detection or quantitation of an analyte suspected of being in a solution comprising: (a) combining said specimen, a first binding component, insoluble particles, and second binding component labeled with a signal generating material in a solid phase retention and separation apparatus having a sufficient pore size such that said particles are trapped within said filter yet permitting rapid passage of fluid therethrough in such a manner that an immunological reaction occurs if analyte is present in said specimen, resulting in the formation of an immunocomplex of insolubilized first binding component:analyte:second labeled binding component on or within said filter means; (b) separating bound from unbound material; and (c) determining the presence and/or amount of signal produced which is correlative with the amount of analyte present in the solution.

### DISCLOSURE OF THE INVENTION

It is an object of the present invention to provide an immunoassay capable of increasing sensitivity in a short reaction time.

It is another object of the present invention to provide an immunoassay capable of achieving satisfactory sensitivity by reacting a ligand with a substance binding specifically to the ligand in a heterogeneous system to form a complex which is a reaction product of the ligand and the specifically binding substance and immobilizing the complex to solid-phase particles through the specifically binding substance to promote the above reaction fully in a short period of time and to provide an immunoassay capable of capturing the ligand immobilized to the solid-phase particles by the above method with a porous fiber matrix, thereby making it possible to ensure the capture and to detect the amount of the ligand easily.

It is a further object of the present invention to provide an immunoassay which enables solid-phase particles for immobilizing the above composite to be prepared without depending on the type of a ligand to be measured and can be used to determine the amounts of various ligands.

It is a still further object of the present invention to provide an immunoassay including a suction mechanism which does require a complicated mechanism such as a pressure sensor or the fine control of suction pressure.

It is a still further object of the present invention to provide an immunoassay including a suction structure capable of capturing a solid-phase immune complex reliably by passing a liquid through a matrix slowly.

It is a still further object of the present invention to provide an immunoassay including a suction mechanism capable of carrying out filtration smoothly without forming a water absorption layer below a filter matrix, thereby making it possible to prevent erroneous detection caused by the immune reaction residue contained in the water absorption layer.

It is a still further object of the present invention to provide an immunoassay in which an immune reaction is not carried out on a filter matrix, thereby making it possible to prevent the non-specific adsorption of an immune reaction component to the filter matrix.

It is a still further object of the present invention to provide an immunoassay reagent used in the above immunoassay of the present invention.

It is a still further object of the present invention to provide an immunological block solution suitable for use in the immunoassay of the present invention.

Other objects and advantages of the present invention will become apparent from the following description.

According to the present invention, firstly, the above objects and advantages of the present invention are attained by an immunoassay for the measurement of a ligand (may be referred to as "second method" hereinafter), comprising the steps of:
(1) contacting a ligand, a first labeled substance binding specifically to said ligand and a second labeled substance binding specifically to said ligand with one another in a solvent to form a complex of said first labeled specifically binding substance, said ligand and said second labeled specifically binding substance;
(2) contacting said complex with carrier solid-phase particles supporting a substance binding specifically to the first labeled substance and having an average particle diameter of 0.3 to 1.0 µm to form a solid-phase complex in which said complex and said carrier solid-phase particles are bound to each other by the first marker;
(3) capturing said solid-phase complex; and
(4) using said solid-phase complex captured matrix for the measurement of the amount of the second marker of said second labeled substance, characterized in that said capturing is carried out with a porous fiber matrix capable of capturing a variety of particle sizes in the inside of said matrix, wherein said variety is the set of particles having a diameter of 0.2-8.0 µm to form a solid-phase complex captured matrix which has said solid-phase complex captured even in the inside of said porous fiber matrix.

According to the present invention, secondly, the above objects and advantages of the present invention are attained by an immunoassay for the measurement of a ligand (may be referred to as "fourth method" hereinafter), comprising the steps of:
(1) contacting a ligand, a first labeled substance binding specifically to said ligand and a second labeled substance binding specifically to said first labeled substance with one another in a solvent to form a mixture of a complex of said first labeled substance and said ligand and a complex of said first labeled substance and said second labeled substance;
(2) contacting said complex mixture with carrier solid-phase particles supporting a substance binding specifically to the labeled substance and having an average particle diameter of 0.3 to 1.0 µm to form a solid-phase complex mixture in which said complex and said carrier solid-phase particles are bound to each other by the first marker;
(3) capturing said solid-phase complex; and
(4) using said solid-phase complex captured matrix for the measurement of the amount of the second marker of said second labeled substance, characterized in that said capturing is carried out with a porous fiber matrix capable of capturing a variety of particle sizes in the inside of said matrix, wherein said variety is the set of particles having a diameter of 0.2-8.0 µm to form a solid-phase complex captured matrix which has said solid-phase complex captured even in the inside of said porous fiber matrix.

According to the present invention, thirdly, the above objects and advantages of the present invention are attained by an immunoassay reagent for the measurement of a ligand, which has a combination of a first labeled substance binding specifically to a ligand, a second labeled substance binding specifically to a ligand, carrier solid-phase particles supporting a substance binding specifically to the first labeled substance and having an average particle diameter of 0.3 to 1.0 µm, characterized in that the combination also comprises a porous fiber matrix capable of capturing a variety of particle sizes in the inside of said matrix, wherein said variety is the set of particles having a diameter of 0.2-8.0 µm and an immunoassay reagent for the measurement of a ligand, which has a combination of a first labeled substance binding specifically to a ligand, a second labeled substance binding specifically to said first labeled substance, carrier solid-phase particles supporting a substance binding specifically to the first labeled substance and having an average particle diameter of 0.3 to 1.0 µm, and a porous fiber matrix capable of capturing a variety of particle sizes in the inside of said matrix, wherein said variety is the set of particles having a diameter of 0.2-8.0 µm.

According to the present invention, in the sixth place, the above objects and advantages of the present invention are attained by the use of an immunological block solution in the above immunoassays which is an aqueous solution having a pH of 7 to 9, containing a buffer having a buffer capacity of pH 7 to 9 and not reacting with casein, nonionic surfactant and calcium ion to form a water-insoluble salt.

### BEST MODE FOR CARRYING OUT THE INVENTION

The present invention will be described in detail hereinunder. A description is first given of a first method, which is not according to the invention. In the first method, a composite of a first labeled specifically binding substance, a ligand and a second labeled specifically binding substance is formed in a homogenous system in the step (1). The ligand may be an antigen or antibody. Examples of the ligand include tumor markers such as AFP, CA19-9, CA125, PSA, ferritin, CEA and CA15-3; hormones such as TSH, T3, T4, LH, FSH, hCG, prolactin, hGH, gastrin, somatostatin, glucagon and insulin; proteins such as IgG, IgM, IgA, IgE, IgD, TBG, CRP and β2-microglobulin; enzymes such as elastase, alkaline phosphatase, amylase, protease, lipase, ribonuclease and enolase; and viruses such as hepatitis virus and AIDS virus, and antibodies to viruses.

A specimen containing a ligand is, for example, a blood fluid such as blood (including serum and plasma), lymph fluid, saliva or urine; or feces or an extracted fluid of a tissue derived from a living body.

The first labeled specifically binding substance and the second labeled specifically binding substance may be the same or different and have specific bindability to a ligand, as exemplified by antibodies, antigens, lectin and protein A.

Examples of the marker of the first labeled specifically binding substance include biotin, avidin, streptoavidin and sugar chains.

Examples of the marker of the second labeled specifically binding substance include isotopes (such as ¹²⁵I), enzymes (such as peroxidase, alkaline phosphatase, β-galactosidase and luciferase), phosphors (such as fluorescein and europium derivatives), and luminous substances (such as acrydinium ester and N-aminobutyl-N-ethyl isoluminol).

The reaction in the step (1) is carried out in a solvent. As the solvent is used a known buffer or a buffer containing a small amount of a surfactant and/or a protein.

The reaction time is preferably 1 to 30 minutes, more preferably 2 to 10 minutes though it differs according to the type of a ligand and the type of a specifically binding substance.

The above composite formed in the step (1) is contacted with carrier solid-phase particles supporting a substance binding specifically to the first marker such as biotin of the first labeled specifically binding substance, for example, an anti-biotin antibody in the step (2). As for the actual operation, the above carrier solid-phase particles are added to the reaction solution after the step (1) and left while they are optionally stirred.

Examples of the solid particles include fine particles of an inorganic substance such as kaolin or carbon; rubber fine particles contained in a natural rubber latex; and polymer fine particles contained in a latex of an organic polymer compound such as polystyrene. The raw material of the above polymer fine particles is, for example, polystyrene, a copolymer of a monomer such as styrene and a monomer having a functional group such as an amino group, thiol group, carboxyl group, active ester group or aldehyde group, or polyacrolein. Specific examples of the raw material include a copolymer of styrene and a phenylmethylsulfonium sulfuric acid salt of methacrylic acid having an active ester group, a copolymer of diethylene glycol dimethacrylate and N-acryloyloxysuccinimide, a copolymer of diethylene glycol dimethacrylate and 1-methacryloyloxybenzotriazole, and polyacrolein having an aldehyde group. The solid-phase particles may be independent particles or agglomerated particles. The average particle diameter of the solid-phase particles is in the range of 0.3 to 1.0 µm. The particle size distribution is preferably narrow. When the average particle diameter of the solid-phase particles is large, the dispersibility in an aqueous solution of the particles lowers and the surface area per weight of the particles decreases, thereby reducing the measurement sensitivity. When the average particle diameter is too small, the capture efficiency of a filter lowers, thereby reducing the final measurement sensitivity.

To support the substance binding specifically to the first marker on the solid-phase particles, conventionally known methods may be used. For example, a method making use of physical adsorption and a method in which solid-phase particles having a functional group on the surface are used to bond the functional group to the functional group of the above substance to be supported by a crosslinkable reagent may be employed.

By the reaction in the step (2), the solid-phase composite in which the above composite is bound to the carrier solid-phase particles by the first marker is formed.

Since the ligand is immobilized to this solid-phase composite in a concentration dependent on its concentration in the specimen, the concentration of the immobilized ligand in the specimen can be determined by measuring the amount of the second marker of the second labeled specifically binding substance in the step (3).

To measure the amount of the second marker contained in the solid-phase composite, a conventionally known method may be used according to the marker. For example, when the marker is an enzyme, (1) a method in which the marker is contacted with a coupling substrate and developed color is measured with a colorimeter making use of an integrating sphere, (2) a method in which the marker is contacted with a fluorescent substrate and fluorescence intensity is measured with a reaction type fluorometer, or (3) a method in which the marker is contacted with a luminous substrate and luminous intensity is measured may be employed. When the marker is a phosphor, fluorescence can be measured by applying suitable excited light to the matrix. When the marker is a luminous substance, emission can be measured by adding a suitable trigger to the luminous substance.

In the above first method, more specifically, (i) the ligand may be an antigen, the first labeled specifically binding substance may be a first labeled antibody to the antigen, and the second labeled specifically binding substance may be a second labeled antibody to the antigen, or the ligand may be an antibody, the first labeled specifically binding substance may be a first labeled antigen to the antibody, and the second labeled specifically binding substance may be a second labeled antigen to the antibody or a second labeled antibody.

In the second method, which is according to the present invention, after the same steps as the steps (1) and (2) of the above first method, next comes the step (3) of forming a solid-phase composite captured matrix by capturing the solid-phase composite formed in the step (2) with the porous fiber matrix.

The solid-phase composite formed in the step (2) consists of a second labeled substance binding specifically to a ligand, the ligand, a first labeled substance binding specifically to the ligand and solid particles supporting a substance binding specifically to the first labeled specifically binding substance all of which are bound to one another in this mentioned order.

Examples of the porous fiber matrix include glass fiber filters, quartz fiber filters, silica fiber filters, nitrocellulose filters, polyacetate filters and filter paper.

Out of these, glass fiber filters are preferred. To prevent non-specific adsorption, the porous fiber matrix may be coated with a suitable protein, sugar or polymer.

Since the porous fiber matrix is composed of a large number of fibers which cross one another 3-dimensionally, holes (spaces) formed by the fibers can capture particles of various sizes. This means that the porous fiber matrix can capture particles of sizes which can be captured in the inside. When the solid-phase composite is captured such that it is distributed in the inside of the porous fiber matrix, the detection of the solid-phase composite can be carried out easily and precisely. The porous fiber matrix used in the present invention has the ability of capturing particles having a diameter of 0.2 to 8.0 µm. Commercially available products of this preferred porous fiber matrix include the AP25 of Millipore Co., Ltd. and the GF/D of Watman Co., Ltd. both of which are made of glass fibers.

This porous fiber matrix is preferably treated with an immunological block solution which is an aqueous solution having a pH of 7 to 9, containing a buffer having a buffer capacity of pH 7 to 9 and not reacting with a casein, nonionic surfactant and calcium ion to form a water-insoluble salt before it is used in the step (3). Examples of the nonionic surfactant include Tween 20, Tween 80, TritonX-100, Noigen 157, octyl glucoside, octyl thioglucoside, heptyl thioglucoside, MEGA-9 and MEGA-10. Preferred examples of the above buffer include Tris-HCl, TES-NaOH, HEPES-NaOH, EPPS-NaOH, Tricine-NaOH and TAPS-NaOH. The above immunological block solution may contain an antiseptic such as NaN3, Micr-O-protect, procline or microcide; a neutral salt such as sodium chloride, magnesium chloride, Glauber's salt or quaternary ammonium salt; a water-soluble polymer such as polyethylene glycol, carboxymethyl cellulose or Ficoll and a sugar such as glucose, sucrose or trehalose. The above immunological block solution has a pH of 7 to 9.

The treatment of the porous fiber matrix with the immunological block solution may be carried out by immersing the porous fiber matrix in the immunological block solution or spraying the immunological block solution over the porous fiber matrix.

The step (3) of capturing the solid-phase composite with the porous fiber matrix is preferably carried out by sucking the solid-phase composite from below the porous fiber matrix to ensure that the filtration rate of the porous fiber matrix becomes 6 to 48 ml/min/cm². The filtration rate is preferably 9.5 to 16 ml/min/cm².

This filtration rate can be attained by installing a continuous porous substance below the porous fiber matrix in the suction direction and sucking the solid-phase composite through the continuous porous substance by a suction pump or forming a space open to the air below the porous fiber matrix in the suction direction and sucking the composite through the space.

The above continuous porous substance is not particularly limited if it can absorb a liquid and has gas permeability. The material of the substance is selected from cellulose, glass, PVA, polyurethane, polyester, polypropylene, vinyl chloride, polyethylene and ceramics.

The porosity of the continuous porous substance is preferably 50 to 95 %, more preferably 80 to 95 %. The diameter of each pore is preferably 20 to 2,000 µm, more preferably 100 to 500 µm. Preferred commercially available products of the continuous porous substance include the Belleater D series Y(D) of Aion Co., Ltd.

Since suction is carried out through the above continuous porous substance, air is sucked in through pores open to the side not in contact with the porous fiber matrix of the continuous porous substance, whereby a liquid can be sucked slowly from the side in contact with the porous fiber matrix through the porous fiber matrix. Suction force which depends on the thickness, porosity and pore diameter of the continuous porous substance or the suction force of the suction pump can be controlled and is desirably controlled by the thickness of the continuous porous substance, that is, the thickness of the continuous porous substance installed between the porous fiber matrix and a suction end extended from the suction pump in most cases.

Alternatively, a space open to the air is formed below the porous fiber matrix in the suction direction so that suction can be carried out through the space. In this case, the porous fiber matrix and the suction end are separated from each other and suction force can be adjusted by the distance between them. By installing the continuous porous substance to surround the space between them, suction force can be adjusted gently. The continuous porous substance in this case may have a cylindrical space formed by scooping out from a portion right below the porous fiber matrix to the suction end.

In the second method, the above solid-phase complex captured matrix formed in the step (3) is measured for the amount of the second marker of the second labeled specifically binding substance in the step (4). This measurement can be carried out in the same manner as in the first method. Actually, a coupling substance or a fluorescent substance is dropped on the solid-phase composite captured matrix according to the second marker to develop a color.

In the third method of the present invention, a so-called "competitive assay" or "competitive inhibition assay" known per se is employed.

In the third method, after the same steps as the steps (1) and (2) of the above first method, next comes the step (3) of forming a solid-phase composite captured matrix by capturing the solid-phase composite mixture formed in the step (2) with the porous fiber matrix. The step (3) may be carried out in the same manner as in the step (3) of the second method.

The solid-phase composite mixture formed in the step (3) of the third method consists of a first solid-phase composite of a ligand, a first labeled substance binding specifically to the ligand and carrier solid-phase particles supporting a substance binding specifically to the first marker of the first labeled specifically binding substance all of which are bound to one another in this order and a second solid-phase composite of a second labeled substance binding specifically to the first labeled specifically binding substance, the first labeled specifically binding substance and the carrier solid-phase particles supporting a substance binding specifically to the first marker of the first labeled specifically binding substance all of which are bound to one another in this order.

As for what is not described of the third method of the present invention, it should be understood that what has been described of the first method and the second method can be applied directly or with modifications obvious to people of ordinary skill in the art.

In the methods of the present invention, a cleaning step may be carried out optionally between steps.

According to the present invention, as understood from the above description, since the substance supported by the carrier solid particles does not bind specifically to the ligand but to the first marker of the first labeled specifically binding substance, the carrier solid particles can be prepared regardless of the type of the ligand. Therefore, according to the present invention, making use of this advantage, there are provided (i) an immunoassay reagent for the measurement of a ligand, which has a combination of a first labeled substance binding specifically to a ligand, a second labeled substance binding specifically to a ligand, carrier solid-phase particles supporting a substance binding specifically to the first labeled substance and having an average particle diameter of 0.3 to 1.0 µm, characterized in that the combination also comprises a porous fiber matrix capable of capturing a variety of particle sizes in the inside of said matrix, wherein said variety is the set of particles having a diameter of 0.2-8.0 µm, and (ii) an immunoassay reagent for the measurement of a ligand, which has a combination of a first labeled substance binding specifically to a ligand, a second labeled substance binding specifically to said first labeled substance, carrier solid-phase particles supporting a substance binding specifically to the first labeled substance and having an average particle diameter of 0.3 to 1.0 µm, and a porous fiber matrix capable of capturing a variety of particle sizes in the inside of said matrix, wherein said variety is the set of particles having a diameter of 0.2-8.0 µm.

Finally, there is provided an immunological block solution which is an aqueous solution having a pH of 7 to 9, containing a buffer having a buffer capacity of pH 7 to 9 and not reacting with casein, nonionic surfactant and calcium ion to form a water-insoluble salt as an immunological block solution advantageously used in the immunoassays of the present invention.

### Examples

The following examples are provided for the purpose of further illustrating the present invention but are in no way to be taken as limiting.

### Example 1

### 1) preparation of a solution containing solid-phase particles supporting an anti-biotin antibody

1 ml of an anti-biotin polyclonal antibody derived from a goat prepared with a phosphoric acid buffer to a concentration of 0.7 mg/ml was added to 1 ml of a solution containing 1.0 % of latex particles (N-500 of Sekisui Chemical Co., Ltd., average particle diameter of 0.5 µm) which had been centrifugally cleaned with a 50 mM phosphoric acid buffer (pH of 7.4) (to be referred to as "phosphoric acid buffer" hereinafter) and shaken at 37°C for 2 hours. Then 1 % bovine serum albumin (BSA) (manufactured by Oriental Enzyme Kogyo Co., Ltd.) prepared with a phosphoric acid buffer was added to the resulting product and further shaken at 37°C for 2 hours. After the supernatant was removed by the centrifugation of the above latex particles supporting an anti-biotin polyclonal antibody, 4 ml of a HEPES buffer containing 0.8 % of NaCl and 1 % of BSA (pH of 8.0) (to be referred to as "HEPES-S buffer" hereinafter) was added to disperse the latex particles. After the supernatant was removed again by the centrifugation of the above latex particles, 4 ml of the HEPES-S buffer was added to disperse the latex particles so as to prepare an anti-biotin antibody supporting solid-phase particle solution.

### 2) preparation of alkaline phosphatase labeled anti-insulin antibody

0.1 ml of a 2-iminothiolan hydrochloride aqueous solution having a concentration of 1 mg/ml was added to 1 ml of a solution containing an anti-insulin monoclonal antibody (OXI005 of Daco Co., Ltd., derived from a mouse) prepared with a phosphoric acid buffer to a concentration of 1 mg/ml and shaken at 25°C for 1 hour. Thereafter, unreacted 2-imonothiolan was removed from the above monoclonal antibody solution by gel filtration to prepare a thiol group introduced anti-insulin antibody. After alkaline phosphatase (manufactured of Kikkoman Corporation) was dissolved in 1 ml of the above thiol group introduced anti-insulin antibody solution having a concentration of 1 mg/ml, 20 µl of N-(γ-maleimidobutyryloxy)succinimide (manufactured by Doj in Kagaku Kenkyusho Co., Ltd.) dissolved in dimethylformamide to a concentration of 5 mg/ml was added to the resulting solution and shaken at 25°C for one night. Thereafter, a fraction in which antibody activity and enzyme activity were seen was dispensed by gel filtration to prepare an alkaline phosphatase labeled anti-insulin antibody.

### 3) preparation of biotin labeled anti-insulin antibody

0.1 ml of Biotin-AC5-OSu (of Dojin Kagaku Kenkyusho Co., Ltd.) dissolved in dimethyl sulfoxide to a concentration of 1 mM was added to 1 ml of a solution containing an anti-insulin monoclonal antibody (HUI018 of Daco Co., Ltd., derived from a mouse) prepared with a 10 mM HEPES buffer (pH of 8.5) to a concentration of 1 mg/ml and left to stand at 25°C for 4 hours. Thereafter, unreacted Biotin-AC5-OSu was removed by gel filtration to prepare a biotin labeled anti-insulin antibody.

### 4) substance to be measured (specimen)

The specimen used for measurement was prepared by diluting commercially available insulin (of Wako Pure Chemical Industries, Ltd.) with a phosphoric acid buffer to a concentration of 0, 0.1, 1, 10 or 100 µIU/ml.

### 5) particle capturing tool

A columnar cup containing a water absorbing material and a glass filler (AP-25 of Millipore Co., Ltd.) was manufactured and used as a latex particle capturing material.

### 6) measurement operation

18 µl of a biotin labeled anti-insulin antibody solution diluted with a phosphoric acid buffer containing 1 % of casein to a concentration of 5 µg/ml, 18 µl of an alkaline phosphatase labeled anti-insulin antibody solution diluted with a phosphoric acid buffer containing 1 % of casein to a concentration of 0.2 µg/ml and 24 µl of a specimen were mixed together and incubated at 37°C for 5 minutes. Thereafter, 20 µl of the anti-biotin antibody supporting solid-phase particle solution prepared in 1) above was added to the above product and incubated at 37°C for 3 minutes to prepare particles to which the alkaline phosphatase labeled anti-insulin antibody was immobilized through the biotin labeled anti-insulin antibody and insulin (to be referred to as "alkaline phosphatase immobilized particles" hereinafter). 50 µl of the prepared alkaline phosphatase immobilized particle solution was dropped on the capturing material wetted out with 50 µl of 25 % Block Ace (of Dainippon Pharmaceutical Co., Ltd.), and 0.1 ml of a phosphoric acid buffer containing 0.05 % of Tween 20 (of Wako Pure Chemical Industries, Ltd.) was dropped on the capturing material twice to clean the glass filter contained in the capturing material Subsequently, this capturing material was used for the measurement of alkaline phosphatase activity.

### 7) measurement of alkaline phosphatase activity

The measurement of the alkaline phosphatase activity contained in the capturing material was carried out with the MI02 fully automatic chemical emission immunoassay (of A & T Co., Ltd.). The APS-5 (of Lumigen Co., Ltd.) was used as a chemical luminous substrate, and 30 µl of APS-5 was dropped on each capturing material.

### 8) measurement results

The relationship between the concentration of insulin and signal intensity obtained by the above measurement is shown in Table 1.

**Table 1**

| Concentration of insulin (µ IU/mL) | Signal intensity |
|---|---|
| 0 | 143 |
| 0.1 | 526 |
| 1 | 4052 |
| 10 | 39382 |
| 100 | 382656 |

### Example 2

The measurement was made in the same manner as in Example 1 except that an anti-PSA monoclonal antibody (10-P20 of Fitzgerald Co., Ltd.) was labeled in place of the alkaline phosphatase labeled anti-insulin antibody, a anti-free PSA monoclonal antibody (10-P21 of Fitzgerald Co., Ltd.) was labeled in place of the biotin labeled anti-insulin antibody, the concentration of the antibody contained in the biotin labeled anti-free PSA monoclonal antibody solution at the time of measurement was changed to 20 µg/ml, the amount of the solution was changed to 10 µl, the concentration of the antibody contained in the alkaline phosphatase labeled anti-PSA monoclonal antibody solution was changed to 2 µg/ml, the amount of the solution was changed to 10 µl, the amount of the specimen was changed to 50 µl, and the specimen was changed to free PSA diluted with a phosphoric acid buffer (concentration: 0, 0.005, 0.05, 0.5 and 5 ng/ml). The results are shown in Table 2.

**Table 2**

| Concentration of free PSA (ng/mL) | Signal intensity |
|---|---|
| 0 | 104 |
| 0.005 | 287 |
| 0.05 | 2992 |
| 0.5 | 27370 |
| 5 | 308974 |

### Example 3

The measurement was made in the same manner as in Example 1 except that an anti-N-ANP monoclonal antibody (7905 of Medics Biochemica Co., Ltd.) was labeled in place of the alkaline phosphatase labeled anti-insulin antibody, an anti-N-ANP monoclonal antibody (7801 of Medics Biochemica Co., Ltd.) was labeled in place of the biotin labeled anti-insulin antibody, the concentration of the antibody contained in the biotin labeled anti-N-ANP monoclonal antibody solution at the time of measurement was changed to 10 µg/ml, the amount of the solution was changed to 15 µl, the concentration of the antibody contained in the alkaline phosphatase labeled anti-N-ANP monoclonal antibody solution was changed to 2 µg/ml, the amount of the solution was changed to 15 µl, the amount of the specimen was changed to 30 µl, and the specimen was changed to N-ANP diluted with a phosphoric acid buffer (concentration: 0,5 50, 500 and 5, 000 pmol/ml). The results are shown in Table 3.

**Table 3**

| Concentration of N-ANP (pmol/L) | Signal intensity |
|---|---|
| 0 | 107 |
| 5 | 307 |
| 50 | 3501 |
| 500 | 33089 |
| 5000 | 322465 |

### Example 4

The measurement was made in the same manner as in Example 1 except that an anti-C-peptide monoclonal antibody (PEP-001 of Daco Co., Ltd.) was labeled in place of the alkaline phosphatase labeled anti-insulin antibody, an anti-C-peptide monoclonal antibody (CPT-3F11 of Daco Co., Ltd.) was labeled in place of the biotin labeled anti-insulin antibody, the concentration of the antibody contained in the biotin labeled anti-C-peptide monoclonal antibody solution at the time of measurement was changed to 5 µg/ml, the amount of the solution was changed to 20 µl, the concentration of the antibody contained in the alkaline phosphatase labeled anti-C-peptide monoclonal antibody solution was changed to 0.2 µg/ml, the amount of the solution was changed to 20 µl, the amount of the specimen was changed to 20 µl, and the specimen was changed to C-peptide diluted with a phosphoric acid buffer (concentration: 0, 0.01, 0.1, 1 and 10 ng/ml). The results are shown in Table 5.

**Table 4**

| Concentration of C-peptide (ng/mL) | Signal intensity |
|---|---|
| 0 | 160 |
| 0.01 | 293 |
| 0.1 | 2698 |
| 1 | 26086 |
| 10 | 255930 |

### Example 5

The measurement, was made in the same manner as in Example 1 except that an anti-pepsinogen I monoclonal antibody (8003 of Medics Biochemica Co., Ltd.) was labeled in place of the alkaline phosphatase labeled anti-insulin antibody, an anti-pepsinogen I monoclonal antibody (8009 of Medics Biochemica Co., Ltd.) was labeled in place of the biotin labeled anti-insulin antibody, the concentration of the antibody contained in the biotin labeled anti-pepsinogen I monoclonal antibody solution at the time of measurement was changed to 10 µg/ml, the amount of the solution was changed to 20 µl, the concentration of the antibody contained in the alkaline phosphatase labeled anti-pepsinogen I monoclonal antibody solution was changed to 0.4 µg/ml, the amount of the solution was changed to 20 µl, the amount of the specimen was changed to 20 µl, and the specimen was changed to pepsinogen I diluted with a phosphoric acid buffer (concentration: 0, 0.1, 2, 16 and 160 ng/ml). The results are shown in Table 5.

**Table 5**

| Concentration of pepsinogen I (ng/mL) | Signal intensity |
|---|---|
| 0 | 98 |
| 0.1 | 263 |
| 2 | 6122 |
| 16 | 40129 |
| 160 | 434310 |

### Example 6

The specimen was measured in the same manner as in Example 1 except that the glass filter contained in the capturing material was changed from AP-25 to GF/D (of Watman Co., Ltd.). The results are shown in Table 6.

**Table 6**

| Concentration of insulin (µIU/mL) | Signal intensity |
|---|---|
| 0 | 207 |
| 0.1 | 731 |
| 1 | 5997 |
| 10 | 55135 |
| 100 | 516586 |

### Example 7

The specimen was measured in the same manner as in Example 1 except that the latex particles supporting an anti-biotin polyclonal antibody were changed to N-300 (of Sekisui Chemical Co., Ltd., average particle diameter of 0.3 µm) and the glass filter contained in the capturing material was changed from AP-25 to AP-15 (of Millipore Co., Ltd.). The results are shown in Table 7.

**Table 7**

| Concentration of insulin (µIU/mL) | Signal intensity |
|---|---|
| 0 | 242 |
| 0.1 | 581 |
| 1 | 5081 |
| 10 | 52354 |
| 100 | 514871 |

### Example 8

### 1) preparation of anti-biotin antibody supporting solid-phase particle solution

1 ml of an anti-biotin polyclonal antibody derived from a goat prepared with a phosphoric acid buffer to a concentration of 0.7 mg/ml was added to 1 ml of a solution containing 1.0 % of latex particles (N-500 of Sekisui Chemical Co., Ltd., average particle diameter of 0.5 µm) which were centrifugally cleaned with a 50 mM phosphoric acid buffer (pH of 7.4) (to be referred to as "phosphoric acid buffer" hereinafter) and shaken at 37°C for 2 hours. Then 1 % bovine serum albumin (BSA) (manufactured by Oriental Enzyme Kogyo Co., Ltd.) prepared with a phosphoric acid buffer was added to the resulting product and further shaken at 37°C for 2 hours. After the supernatant was removed by the centrifugation of the above latex particles supporting an anti-biotin polyclonal antibody, 4 ml of a HEPES buffer containing 0.8 % of NaCl and 1 % of BSA (pH of 8.0) (to be referred to as "HEPES-S buffer" hereinafter) was added to disperse the latex particles. After the supernatant was removed again by the centrifugation of the latex particles, 4 ml of the HEPES-S buffer was added to disperse the latex particles so as to prepare an anti-biotin antibody supporting solid-phase particle solution.

### 2) preparation of alkaline phosphatase labeled anti-C-peptide antibody

0.1 ml of a 2-iminothiolan hydrochloride aqueous solution having a concentration of 1 mg/ml was added to 1 ml of a solution containing an anti-C-peptide monoclonal antibody (PEP001 of Daco Co., Ltd., derived from a mouse) prepared with a phosphoric acid buffer to a concentration of 1 mg/ml and shaken at 25°C for 1 hour. Thereafter, unreacted 2-imonothiolan was removed from the above monoclonal antibody solution by gel filtration to prepare a thiol group introduced anti-insulin antibody. After alkaline phosphatase (of Kikkoman Corporation) was dissolved in 1 ml of the above thiol group introduced anti-insulin antibody solution having a concentration of 1 mg/ml, 20 µl of N-(γ-maleimidobutyryloxy)succinimide (manufactured by Dojin Kagaku Kenkyusho Co., Ltd.) dissolved in dimethylformamide to a concentration of 5 mg/ml was added and shaken at 25°C for one night. Thereafter, a fraction in which antibody activity and enzyme activity were seen was dispensed by gel filtration to prepare an alkaline phosphatase labeled anti-C-peptide antibody.

### 3) preparation of biotin labeled anti-C-peptide antibody

0.1 ml of Biotin-AC5-OSu (of Dojin Kagaku Kenkyusho Co., Ltd.) dissolved in dimethyl sulfoxide to a concentration of 1 mM was added to 1 ml of a solution containing an anti-C-peptide monoclonal antibody (CPT3F11 of Daco Co., Ltd., derived from a mouse) prepared with a 10 mM HEPES buffer (pH of 8.5) to a concentration of 1 mg/ml and left to stand at 25°C for 4 hours. Thereafter, unreacted Biotin-AC5-OSu was removed by gel filtration to prepare a biotin labeled anti-C-peptide antibody.

### 4) substance to be measured (specimen)

The specimen used for measurement was prepared by diluting commercially available C-peptide (manufactured by Cosmo Bio Co., Ltd.) with a phosphoric acid buffer to a concentration of 0, 0.1 or 3 ng/ml.

### 5) particle capturing tool

A columnar cup containing a water absorbing material and a glass filler (AP-25 of Millipore Co., Ltd.) was manufactured and used as a latex particle capturing material.

### 6) measurement operation

20 µl of a biotin labeled anti-C-peptide antibody solution diluted with a phosphoric acid buffer containing 1 % of casein to a concentration of 5 µg/ml, 10 µl of an alkaline phosphatase labeled anti-C-peptide antibody solution diluted with a phosphoric acid buffer containing 1 % of casein to a concentration of 0.1 µg/ml and 10 µl of a specimen were mixed together and incubated at 37°C for 10 minutes. Thereafter, 20 µl of the anti-biotin antibody supporting solid-phase particle solution prepared in 1) above was added to the above product and incubated at 37°C for 3 minutes to prepare particles to which the alkaline phosphatase labeled anti-C-peptide antibody was immobilized through the biotin labeled anti-C-peptide antibody and C-peptide (to be referred to as "alkaline phosphatase immobilized particles" hereinafter). 50 µl of the prepared alkaline phosphatase immobilized particle solution was dropped on the capturing material wetted out with 50 µl of a block solution which was a trishydrochloric acid buffer (pH of 7.4) containing 1 % of casein (manufactured by Wako Pure Chemical Industries, Ltd.), 0.1 % of Triton X-100 and 0.8 % of NaCl, and 0.1 ml of a phosphoric acid buffer containing 0.05 % of Tween 20 (of Wako Pure Chemical Industries, Ltd.) was dropped on the capturing material twice to clean the glass filter contained in the capturing material. Subsequently, this capturing material was used for the measurement of alkaline phosphatase activity. The above measurement operation was made twice on the same specimen.

### 7) measurement of alkaline phosphatase activity

The measurement of alkaline phosphatase activity in the capturing material was carried out with the MI02 fully automatic chemical emission immunoassay (of A & T Co., Ltd.) . APS-5 (of Lumigen Co., Ltd.) was used as a chemical luminous substrate, and 30 µl of APS-5 was dropped on each capturing material.

### 8) measurement results

The relationship between the concentration of C-peptide and signal intensity obtained by the above measurement is shown in Table 8.

**Table 8**

| Concentration of C-peptide (ng/mL) | Signal intensity |
|---|---|
| 0 | 189 |
| 0 | 181 |
| 0.1 | 574 |
| 0.1 | 595 |
| 3 | 10923 |
| 3 | 10966 |

### Example 9

### 1) preparation of anti-biotin antibody supporting solid-phase particle solution

1 ml of an anti-biotin polyclonal antibody derived from a goat prepared with a phosphoric acid buffer to a concentration of 0.7 mg/ml was added to 1 ml of a solution containing 1.0 % of latex particles (N-500 of Sekisui Chemical Co., Ltd., average particle diameter of 0.5 µm) which were centrifugally cleaned with a phosphoric acid buffer (pH of 7.4) having a concentration of 0.05 mol/l (to be referred to as "phosphoric acid buffer" hereinafter) and shaken at 37°C for 2 hours. Then 1 ml of 1 % bovine serum albumin (BSA) (manufactured by Oriental Enzyme Kogyo Co., Ltd.) prepared with a phosphoric acid buffer was added to the resulting product and further shaken at 37°C for 2 hours. After the supernatant was removed by the centrifugation of the above latex particles supporting an anti-biotin polyclonal antibody, 4 ml of a HEPES buffer containing 0.8 % of NaCl and 1 % of BSA (pH of 8.0) (to be referred to as "HEPES-S buffer" hereinafter) was added to disperse the latex particles. After the supernatant was removed again by the centrifugation of the latex particles, 4 ml of the HEPES-S buffer was added to disperse the latex particles so as to prepare an anti-biotin antibody supporting solid-phase particle solution.

### 2) preparation of alkaline phosphatase labeled anti-C-peptide antibody

0.1 ml of a 2-iminothiolan hydrochloride aqueous solution having a concentration of 1 mg/ml was added to 1 ml of a solution containing an anti-C-peptide monoclonal antibody (PEP-001 of Daco Co., Ltd., derived from a mouse) prepared with a phosphoric acid buffer to a concentration of 1 mg/ml and shaken at 25°C for 1 hour. Thereafter, unreacted 2-imonothiolan was removed from the above monoclonal antibody solution by gel filtration to prepare a thiol group introduced anti-C-peptide antibody. After alkaline phosphatase (manufactured by Kikkoman Corporation) was dissolved in 1 ml of the above thiol group introduced anti-C-peptide antibody solution having a concentration of 1 mg/ml, 20 µl of N-(γ-maleimidobutyryloxy)succinimide (manufactured by Doj in Kagaku Kenkyusho Co. , Ltd.) dissolved in dimethylformamide to a concentration of 5 mg/ml was added to the resulting product and shaken at 25°C for one night. Thereafter, a fraction in which antibody activity and enzyme activity were seen was dispensed by gel filtration to prepare an alkaline phosphatase labeled anti-C-peptide antibody.

### 3) preparation of biotin labeled anti-C-peptide antibody

0.1 ml of Biotin-AC5-OSu (of Dojin Kagaku Kenkyusho Co., Ltd.) dissolved in dimethyl sulfoxide to a concentration of 0.001 mol/l was added to 1 ml of a solution containing an anti-C-peptide monoclonal antibody (CPT-3-F11 of Daco Co., Ltd., derived from a mouse) prepared with a 0.01 mol/l HEPES buffer (pH of 8.5) to a concentration of 1 mg/ml and left to stand at 25°C for 4 hours. Thereafter, unreacted Biotin-AC5-OSu was removed by gel filtration to prepare a biotin labeled anti-C-peptide antibody.

### 4) substance to be measured (specimen)

The specimen used for measurement was prepared by diluting commercially available C-peptide (manufactured by Cosmo Bio Co., Ltd.) with a phosphoric acid buffer to a concentration of 0, 0.1 or 3 ng/ml.

### 5) particle capturing tool

A columnar cup having a 6 mm-diameter hole at the bottom and a glass filter (AP-25 of Millipore Co., Ltd.) placed at the bottom was manufactured and used as a latex particle capturing material.

### 6) suction and discharge apparatus

A 1-cm cubic PVA sponge (Belleater D series Y(D) of Aion Co., Ltd.) was attached to one end of a suction nozzle (stainless pipe having an inner diameter of 2.5 mm) and installed below the hole of the above columnar cup having a glass filter. A pressure tube was attached to the other end of the stainless pipe and connected to a vacuum pump (VP0125 of Medo Sangyo Co., Ltd., air delivery rate: 7 1/min) through a discharge trap to construct a suction and discharge apparatus. The filtration rate of the glass filter used in the capturing material 5) above when the apparatus was used was 12 ml/min/cm².

### 7) measurement operation

20 µl of a biotin labeled anti-C-peptide antibody solution diluted with a phosphoric acid buffer containing 1 % of casein to a concentration of 5 µg/ml, 10 µl of an alkaline phosphatase labeled anti-C-peptide antibody solution diluted with a phosphoric acid buffer containing 1 % of casein to a concentration of 0.1 µg/ml and 10 µl of a specimen were mixed together and incubated at 37°C for 10 minutes. Thereafter, 20 µl of the anti-biotin antibody supporting solid-phase particle solution prepared in 1) above was added to the above product and further incubated at 37°C for 3 minutes to prepare particles to which the alkaline phosphatase labeled anti-C-peptide antibody was immobilized through the biotin labeled anti-C-peptide antibody and C-peptide (to be referred to as "alkaline phosphatase immobilized particles" hereinafter). The capturing material prepared in 5) above was placed on the PVA sponge of the suction and discharge apparatus constructed in 6) above. 30 µl of the prepared alkaline phosphatase immobilized particle solution was dropped on the capturing material wetted out with 50 µl of a block solution which was a trishydrochloric acid buffer (pH of 7.4) containing 0.1 % of casein (manufactured by Wako Pure Chemical Industries, Ltd.), 0.1 % of Triton X-100 and 0.8 % of NaCl, and 0.1 ml of a phosphoric acid buffer containing 0.05 % of Tween 20 (of Wako Pure Chemical Industries, Ltd.) was dropped on the capturing material twice to clean the glass filter contained in the capturing material. Subsequently, this capturing material was used for the measurement of alkaline phosphatase activity. The above measurement operation was made twice on the same specimen.

### 7) measurement of alkaline phosphatase activity

The measurement of alkaline phosphatase activity in the capturing material was carried out by using a photomultiplier module (H7360-02 of Hamamatsu Photonics K.K.) in a dark place. APS-5 (of Lumigen Co., Ltd.) was used as a chemical luminous substrate, and 30 µl of APS-5 was dropped on each capturing material. One minute after dropping, signal intensity was measured every 0.1 second 10 times to calculate the average of the measurement data as a measurement value.

### 9) measurement results

The concentration of C-peptide and signal intensity obtained by the above measurement is shown in Table 9.

**Table 9**

| Concentration of C-peptide (ng/mL) | Signal intensity |
|---|---|
| 0 | 1038 |
| 0 | 1026 |
| 0.1 | 5079 |
| 0.1 | 5083 |
| 3 | 111293 |
| 3 | 111353 |

As described above, according to the immunoassay and reagent of the present invention, the amount of a ligand to be measured can be determined at a high repeatability and a high sensitivity in a short period of time.

According to the preferred immunoassay of the present invention, a complicated mechanism such as a pressure sensor and the fine control of suction pressure are not required to capture a solid-phase complex with a porous fiber matrix, and a liquid passes through the porous fiber matrix slowly, thereby making it possible to adsorb the solid-phase composite particles surely. Further, a water absorbing layer containing the reaction residue does not need to be placed below the porous fiber matrix, thereby making it possible to prevent erroneous detection. Further, as a immune reaction is not carried out on the porous fiber matrix for a long time, the non-specific adsorption to the matrix of the reaction component can be suppressed, thereby making it possible to determine the amount of the ligand to be measured at a high repeatability and a high sensitivity in a short period of time.

## Claims

1. An immunoassay for the measurement of a ligand, comprising the steps of:
(1) contacting a ligand, a first labeled substance binding specifically to said ligand and a second labeled substance binding specifically to said ligand with one another in a solvent to form a complex of said first labeled specifically binding substance, said ligand and said second labeled specifically binding substance;
(2) contacting said complex with carrier solid-phase particles supporting a substance binding specifically to the first labeled substance and having an average particle diameter of 0.3 to 1.0 µm to form a solid-phase complex in which said complex and said carrier solid-phase particles are bound to each other by the first marker;
(3) capturing said solid-phase complex; and
(4) using said solid-phase complex captured matrix for the measurement of the amount of the second marker of said second labeled substance, **characterized in that** said capturing is carried out with a porous fiber matrix capable of capturing a variety of particle sizes in the inside of said matrix, wherein said variety is the set of particles having a diameter of 0.2-8.0 µm to form a solid-phase complex captured matrix which has said solid-phase complex captured even in the inside of said porous fiber matrix.

2. The immunoassay according to claim 1, wherein said ligand is an antigen, said first labeled substance is a first labeled antibody to said antigen, and said second labeled substance is a second labeled antibody to said antigen.

3. The immunoassay according to claim 1, wherein said ligand is an antibody, said first labeled substance is a first labeled antigen to said antibody, and said second labeled substance is a second labeled antigen to said antibody or a second labeled antibody.

4. The immunoassay according to claim 1, wherein said porous fiber matrix is treated prior to step (3) with a block solution which is an aqueous solution having pH of 7 to 9, containing a buffer having a buffer capacity of pH 7 to 9 and not reacting with casein, a nonionic surfactant and calcium ion to form a water-insoluble salt.

5. The immunoassay according to claim 1, wherein said solid-phase complex captured matrix is formed by sucking from below said porous fiber matrix at a filtration rate of 6 to 48 ml/min/cm².

6. An immunoassay for the measurement of a ligand, comprising the steps of:
(1) contacting a ligand, a first labeled substance binding specifically to said ligand and a second labeled substance binding specifically to said first labeled substance with one another in a solvent to form a mixture of a complex of said first labeled substance and said ligand and a complex of said first labeled substance and said second labeled substance;
(2) contacting said complex mixture with carrier solid-phase particles supporting a substance binding specifically to the labeled substance and having an average particle diameter of 0.3 to 1.0 µm to form a solid-phase complex mixture in which said complex and said carrier solid-phase particles are bound to each other by the first marker;
(3) capturing said solid-phase complex; and
(4) using said solid-phase complex captured matrix for the measurement of the amount of the second marker of said second labeled substance, **characterized in that** said capturing is carried out with a porous fiber matrix capable of capturing a variety of particle sizes in the inside of said matrix, wherein said variety is the set of particles having a diameter of 0.2-8.0 µm to form a solid-phase complex captured matrix which has said solid-phase complex captured even in the inside of said porous fiber matrix.

7. The immunoassay according to claim 6, wherein said ligand is an antigen, said first labeled substance is a first labeled antibody to said antigen, and said second labeled substance is the labeled antigen.

8. The immunoassay according to claim 6, wherein said ligand is an antibody said first labeled substance is a first labeled antigen to said antibody, and said second labeled substance is the labeled antibody.

9. The immunoassay according to claim 6, wherein said porous fiber matrix is treated prior to step (3) with a block solution which is an aqueous solution having pH of 7 to 9, containing a buffer having a buffer capacity of pH 7 to 9 and not reacting with casein, a nonionic surfactant and calcium ion to form a water-insoluble salt.

10. The immunoassay according to claim 6, wherein said solid-phase complex captured matrix is formed by sucking from below said porous fiber matrix at a filtration rate of 6 to 48 ml/min/cm².

11. An immunoassay reagent for the measurement of a ligand, which has a combination of a first labeled substance binding specifically to a ligand, a second labeled substance binding specifically to a ligand, carrier solid-phase particles supporting a substance binding specifically to the first labeled substance and having an average particle diameter of 0.3 to 1.0 µm, **characterized in that** the combination also comprises a porous fiber matrix capable of capturing a variety of particle sizes in the inside of said matrix, wherein said variety is the set of particles having a diameter of 0.2-8.0 µm.

12. An immunoassay reagent for the measurement of a ligand, which has a combination of a first labeled substance binding specifically to a ligand, a second labeled substance binding specifically to said first labeled substance, carrier solid-phase particles supporting a substance binding specifically to the first labeled substance and having an average particle diameter of 0.3 to 1.0 µm, and a porous fiber matrix capable of capturing a variety of particle sizes in the inside of said matrix, wherein said variety is the set of particles having a diameter of 0.2-8.0 µm.

## Patentansprüche

1. Immunoassay zur Messung eines Liganden, umfassend die folgenden Stufen:
(1) Inkontaktbringen eines Liganden, einer ersten markierten Substanz, die spezifisch an den Liganden bindet, und einer zweiten markierten Substanz, die spezifisch an den Liganden bindet, miteinander in einem Lösungsmittel unter Bildung eines Komplexes der ersten markierten spezifisch bindenden Substanz, des Liganden und der zweiten markierten spezifisch bindenden Substanz;
(2) Inkontaktbringen des Komplexes mit Träger-Festphasenpartikeln, die eine Substanzbindung spezifisch an die erste markierte Substanz unterstützen und einen durchschnittlichen Partikeldurchmesser von 0,3 bis 1,0 µm aufweisen, unter Bildung eines Festphasenkomplexes, in welchem der Komplex und die Träger-Festphasenpartikel über den ersten Marker aneinander gebunden sind;
(3) Fangen des Festphasenkomplexes; und
(4) Verwenden der Matrix mit dem gefangenen Festphasenkomplex zur Messung der Menge des zweiten Markers der zweiten markierten Substanz, **dadurch gekennzeichnet, dass** das Fangen mit einer porösen Fasermatrix durchgeführt wird, die zum Fangen einer Vielfalt von Partikelgrößen im Inneren der Matrix befähigt ist, wobei die Vielfalt die Gruppe von Partikeln mit einem Durchmesser von 0,2 bis 8,0 µm ist, unter Bildung einer Matrix mit dem gefangenen Festphasenkomplex, welche den Festphasenkomplex gleichmäßig gefangen im Inneren der porösen Fasermatrix aufweist.

2. Immunoassay gemäß Anspruch 1, wobei der Ligand ein Antigen ist, die erste markierte Substanz ein erster markierter Antikörper für das Antigen ist und die zweite markierte Substanz ein zweiter markierter Antikörper für das Antigen ist.

3. Immunoassay gemäß Anspruch 1, wobei der Ligand ein Antikörper ist, die erste markierte Substanz ein erstes markiertes Antigen für den Antikörper ist und die zweite markierte Substanz ein zweites markiertes Antigen für den Antikörper oder ein zweiter markierter Antikörper ist.

4. Immunoassay gemäß Anspruch 1, wobei die poröse Fasermatrix vor Stufe (3) mit einer Blockierungslösung behandelt wird, welche eine wässrige Lösung mit einem pH von 7 bis 9 ist, die einen Puffer, der eine Pufferkapazität von pH 7 bis 9 aufweist und nicht mit Casein reagiert, ein nichtionisches oberflächenaktives Mittel und Calciumionen zur Bildung eines wasserunlöslichen Salzes enthält.

5. Immunoassay gemäß Anspruch 1, wobei die Matrix mit dem gefangenen Festphasenkomplex durch Saugen von unterhalb der porösen Fasermatrix mit einer Filtrationsgeschwindigkeit von 6 bis 48 ml/min/cm² gebildet wird.

6. Immunoassay zur Messung eines Liganden, umfassend die Stufen:
(1) Inkontaktbringen eines Liganden, einer ersten markierten Substanz, die spezifisch an den Liganden bindet, und einer zweiten markierten Substanz, die spezifisch an die erste markierte Substanz bindet, miteinander in einem Lösungsmittel unter Bildung eines Komplexes der ersten markierten Substanz und des Liganden und eines Komplexes der ersten markierten Substanz und der zweiten markierten Substanz;
(2) Inkontaktbringen des Komplexgemischs mit Träger-Festphasenpartikeln, die eine Substanzbindung spezifisch an die markierte Substanz unterstützen und einen durchschnittlichen Partikeldurchmesser von 0,3 bis 1,0 µm haben, unter Bildung eines Festphasenkomplexgemisches, in welchem der Komplex und die Träger-Festphasenpartikel über den ersten Marker aneinander gebunden sind;
(3) Fangen des Festphasenkomplexes; und
(4) Verwenden der Matrix mit dem gefangenen Festphasenkomplex zur Messung der Menge des zweiten Markers der zweiten markierten Substanz, **dadurch gekennzeichnet, dass** das Fangen mit einer porösen Fasermatrix durchgeführt wird, die zum Fangen einer Vielfalt von Partikelgrößen im Inneren der Matrix befähigt ist, wobei die Vielfalt die Gruppe von Partikeln mit einem Durchmesser von 0,2 bis 8,0 µm ist, unter Bildung einer Matrix mit dem gefangenen Festphasenkomplex, welche den Festphasenkomplex gleichmäßig gefangen im Inneren der porösen Fasermatrix aufweist.

7. Immunoassay gemäß Anspruch 6, wobei der Ligand ein Antigen ist, die erste markierte Substanz ein erster markierter Antikörper für das Antigen ist und die zweite markierte Substanz das markierte Antigen ist.

8. Immunoassay gemäß Anspruch 6, wobei der Ligand ein Antikörper ist, die erste markierte Substanz ein erstes markiertes Antigen für den Antikörper ist und die zweite markierte Substanz der markierte Antikörper ist.

9. Immunoassay gemäß Anspruch 6, wobei die poröse Fasermatrix vor Stufe (3) mit einer Blockierungslösung behandelt wird, welche eine wässrige Lösung mit einem pH von 7 bis 9 ist, die einen Puffer, der eine Pufferkapazität von pH 7 bis 9 aufweist und nicht mit Casein reagiert, ein nichtionisches oberflächenaktives Mittel und Calciumionen zur Bildung eines wasserunlöslichen Salzes enthält.

10. Immunoassay gemäß Anspruch 6, wobei die Matrix mit dem gefangenen Festphasenkomplex durch Saugen von unterhalb der porösen Fasermatrix mit einer Filtrationsgeschwindigkeit von 6 bis 48 ml/min/cm² gebildet wird.

11. Immunoassayreagens zur Messung eines Liganden, welches eine Kombination aus einer ersten markierten Substanz, die spezifisch an einen Liganden bindet, einer zweiten markierten Substanz, die spezifisch an einen Liganden bindet, Träger-Festphasenpartikeln; die eine Substanzbindung spezifisch an die erste markierte Substanz unterstützen und einen durchschnittlichen Partikeldurchmesser von 0,3 bis 1,0 µm aufweisen, aufweist, **dadurch gekennzeichnet, dass** die Kombination auch eine poröse Fasermatrix umfasst, die zum Fangen einer Vielfalt von Partikelgrößen im Inneren der Matrix befähigt ist, wobei die Vielfalt eine Gruppe von Partikeln mit einem Durchmesser von 0,2 bis 8,0 µm ist.

12. Immunoassayreagens zur Messung eines Liganden, welches eine Kombination aus einer ersten markierten Substanz, die spezifisch an einen Liganden bindet, einer zweiten markierten Substanz, die spezifisch an die erste markierte Substanz bindet, Träger-Festphasenpartikeln, die eine Substanzbindung spezifisch an die erste markierte Substanz unterstützen und einen durchschnittlichen Partikeldurchmesser von 0,3 bis 1,0 µm aufweisen, und eine poröse Fasermatrix, die zum Fangen einer Vielfalt von Partikelgrößen im Inneren der Matrix befähigt ist, wobei die Vielfalt die Gruppe von Partikeln mit einem Durchmesser von 0,2 bis 8,0 µm ist, aufweist.

## Revendications

1. Dosage immunologique pour mesurer un ligand, comprenant les étapes suivantes:
(1) mettre en contact entre eux un ligand, une première substance marquée, se liant spécifiquement audit ligand, et une seconde substance marquée, se liant spécifiquement audit ligand, dans un solvant de façon à former un complexe de ladite première substance marquée liante spécifiquement, ledit ligand et ladite seconde substance marquée liant spécifiquement;
(2) mettre en contact ledit complexe avec des particules support en phase solide supportant une substance se liant spécifiquement à la première substance marquée et ayant un diamètre de particule moyen de 0,3 à 1,0 µm de façon à former un complexe en phase solide dans lequel le complexe est lié aux particules support en phase solide et lesdites particules en phase solide par le marqueur premier;
(3) capturer ledit complexe en phase solide; et
(4) utiliser ladite matrice capturée en phase solide pour la détermination de la quantité du second marqueur de ladite seconde substance marquée,
**caractérisé en ce que** ladite capture est effectuée par une matrice fibreuse poreuse capable de capturer une gamme de particules à l'intérieur de ladite matrice, ladite gamme étant l'ensemble de particules ayant un diamètre de 0,2-8,0 µm, de façon à former une matrice capturée en complexe en phase solide dans laquelle ledit complexe en phase solide est même capturé à l'intérieur de ladite matrice fibreuse poreuse.

2. Dosage immunologique selon la revendication 1, **caractérisé en ce que** le ligand est un antigène, ladite première substance marquée est un premier anticorps marqué contre ledit antigène et ladite seconde substance marquée est un second anticorps marqué contre ledit antigène.

3. Dosage immunologique selon la revendication 1, **caractérisé en ce que** le ligand est un anticorps, ladite première substance marquée est un premier antigène marqué contre ledit anticorps et ladite seconde substance marquée est un second antigène marqué contre ledit anticorps ou un second anticorps marquée.

4. Dosage immunologique selon la revendication 1, **caractérisé en ce que** ladite matrice fibreuse poreuse est traitée avant l'étape (3) avec une solution bloquante, étant une solution aqueuse avec un pH de 7 à pH 9, contenant un tampon avec une capacité de tampon de pH 7 à pH 9, étant non-réactif avec la caséine, un agent tensio-actif non-ionique et un ion calcium de façon à former un sel insoluble dans l'eau.

5. Dosage immunologique selon la revendication 1, **caractérisé en ce que** ladite matrice capturée en complexe en phase solide est formée par aspiration au-dessous de ladite matrice fibreuse poreuse à une vitesse de filtration de 6 à 48 ml/cm².

6. Dosage immunologique pour mesurer un ligand, comprenant les étapes suivantes:
(1) mettre en contact entre eux un ligand, une première substance marquée, se liant spécifiquement audit ligand, et une seconde substance marquée, se liant spécifiquement à la première substance marquée, dans un solvant de façon à former un mélange d'un complexe de ladite première substance marquée et ledit ligand et un complexe de ladite première substance marquée et ladite seconde substance marquée;
(2) mettre en contact ledit mélange de complexes avec des particules support en phase solide supportant une substance se liant spécifiquement à la substance marquée et ayant un diamètre de particule moyen de 0,3 à 1,0 µm de façon à former un complexe en phase solide dans lequel le complexe est lié aux particules support en phase solide et lesdites particules en phase solide par le marqueur premier;
(3) capturer ledit complexe en phase solide; et
(4) utiliser ladite matrice capturée en phase solide pour la détermination de la quantité du second marqueur de ladite seconde substance marquée,
**caractérisé en ce que** ladite capture est effectuée par une matrice fibreuse poreuse capable de capturer une gamme de particules à l'intérieur de ladite matrice, ladite gamme étant l'ensemble de particules ayant un diamètre de 0,2-8,0 µm, de façon à former une matrice capturée en complexe en phase solide dans laquelle ledit complexe en phase solide est même capturé à l'intérieur de ladite matrice fibreuse poreuse.

7. Dosage immunologique selon la revendication 6, **caractérisé en ce que** le ligand est un antigène, ladite première substance marquée est un premier anticorps marqué contre ledit antigène et ladite seconde substance marquée est l'antigène marqué.

8. Dosage immunologique selon la revendication 6, **caractérisé en ce que** le ligand est un anticorps, ladite première substance marquée est un premier antigène marqué contre ledit anticorps et ladite seconde substance est l'anticorps marqué.

9. Dosage immunologique selon la revendication 6, **caractérisé en ce que** ladite matrice fibreuse poreuse est traitée avant l'étape (3) avec une solution bloquante, étant une solution aqueuse avec un pH de 7 à pH 9, contenant un tampon avec une capacité de tampon de pH 7 à pH 9, étant non-réactif avec la caséine, un agent tensio-actif non-ionique et un ion calcium de façon à former un sel insoluble dans l'eau.

10. Dosage immunologique selon la revendication 6, **caractérisé en ce que** ladite matrice capturée en complexe en phase solide est formée par aspiration au-dessous de ladite matrice fibreuse poreuse à une vitesse de filtration de 6 à 48 ml/cm².

11. Réactif pour dosage immunologique pour la mesure d'un ligand, ayant une combinaison d'une première substance marquée se liant spécifiquement à un ligand, une seconde substance marquée se liant spécifiquement à un ligand, des particules support en phase solide supportant une substance se liant spécifiquement à la première substance marquée et ayant un diamètre de particules moyen de 0,3 à 1,0 µm, **caractérisé en ce que** la combinaison comprend en outre une matrice fibreuse poreuse capable de capturer une gamme de particules à l'intérieur de ladite matrice, ladite gamme étant l'ensemble de particules ayant un diamètre de 0,2 à 8,0 µm.

12. Réactif pour dosage immunologique pour la mesure d'un ligand, ayant une combinaison d'une première substance marquée, se liant spécifiquement à un ligand, une seconde substance marquée, se liant spécifiquement à ladite première substance marquée, des particules support en phase solide supportant une substance se liant spécifiquement à la première substance marquée et ayant un diamètre de particules moyen de 0,3 à 1,0 µm, et une matrice fibreuse poreuse, capable de capturer une gamme de particules à l'intérieur de ladite matrice, ladite gamme étant l'ensemble de particules ayant un diamètre de 0,2-8,0 µm.
